# EUROPÄISCHE PATENTSCHRIFT

(11) **EP 0 408 946 B1**
(45) Veröffentlichungstag und Bekanntmachung des Hinweises auf die Patenterteilung: **23.03.1994**
(21) Anmeldenummer: 90112403.2
(22) Anmeldetag: 29.06.1990
(51) Int. Cl.: A61L 2/12

(54) **Verfahren und Vorrichtung zum Keimfreimachen, Desinfizieren und/oder Trocknen**
Method and apparatus for sterilising, disinfecting and/or drying
Méthode et dispositif pour la stérilisation, la désinfection et/ou le séchage

(30) Priorität: 19.07.1989 DE 3923841
(43) Veröffentlichungstag der Anmeldung: 23.01.1991
(73) Patentinhaber: Satow, Kurt, D-28816 Stuhr (DE)
(72) Erfinder: Satow, Kurt, D-2820 Bremen Stuhr (DE); Hoffmann, Manfred, D-2850 Bremerhaven (DE); Göldner, Helmut, D-3070 Nienburg (DE)
(74) Vertreter: Eisenführ, Speiser & Partner

(56) Entgegenhaltungen:
- EP-A- 0 320 193
- WO-A-86/02842
- FR-A- 2 240 041
- US-A- 3 670 891
- PATENT ABSTRACTS OF JAPAN, vol. 012, no. 037 (C-473), 4. Februar 1988; & JP-A-62 186 770

## Beschreibung

Die Erfindung betrifft eine Vorrichtung zum Keimfreimachen, Desinfizieren und/oder Trocknen von schüttfähigen Stoffen in loser Form in einem geschlossenen, dichten Behälter unter Anwendung von Druck oder Vakuum zusammen mit Mikrowellenenergie und unter Umwälzung der Stoffe in dem Behälter.

Es ist bereits bekannt, Krankenhausmüll durch Beheizung von Mikrowellen zu desinfizieren. Eine solche Anlage ist in einem Prospekt der Firma Vetco Sanitec GmbH beschrieben. Der Krankenhausmüll wird bei einer solchen Anlage zunächst mechanisch zerkleinert, anschließend befeuchtet und dann mittels einer Förderschnecke an Mikrowelleneinheiten vorbeigeführt, um durch schnelles, gezieltes Aufheizen bis zum Siedepunkt des Wassers den Müll zu desinfizieren und in dieser zerkleinerten Form zur Deponie auszustoßen. Es ist klar, daß das zu desinfizierende Gut hier nicht schonend behandelt werden muß, da es ja anschließend auf einer Mülldeponie oder dergleichen landet, so daß die gewünschte Desinfektion durch starke Zerkleinerung und hohe Temperaturen stets erreicht werden kann.

Sollen jedoch schüttfähige Stoffe in loser Form schonend keimfrei gemacht oder getrocknet werden, ohne daß die Eigenschaften und die Qualität dieser Stoffe beeinträchtigt werden, so ist das bekannte Verfahren wegen der notwendigen Zerkleinerung und hohen Temperaturen meist nicht anwendbar.

Aus der DE-OS 36 12 606 ist ein Verfahren zum Entkeimen bzw. Desinfizieren von pharmazeutischen Zubereitungen bekannt, bei dem die Behandlung in geschlossenen Ampullen mit Hilfe von Mikrowellen bei einer für eine Sterilisation notwendigen Temperatur durchgeführt wird. Aufgrund dieser Temperaturerhöhung entsteht in den Ampullen jedoch ein Überdruck, so daß hier die Gefahr eines Zerstörens besteht. Aus diesem Grunde wird bei diesen bekannten Verfahren vorgeschlagen, die Mikrowellenbehandlung von in Ampullen befindlichen Stoffen in einem Druckbehälter unter Überdruck vorzunehmen, damit die Ampullen nicht zerplatzen können.

Aus der DE-OS 24 46 471 ist ein Verfahren zum Trocknen und Sterilisieren mittels Mikrowellen bekannt, bei dem das zu behandelnde Gut ebenfalls in Ampullen untergebracht ist. Um die Trocknungswirkung zu vergrößern, erfolgt die Behandlung in einem geschlossenen Behälter unter Anwendung von laufenden Druckwechseln, und zwar beginnend mit atmosphärischem Druck, anschließendem Vakuum, anschließendem Überdruck usw. Hierdurch sollen in dem zu behandelnden Gut enthaltende Dämpfe in einem geschlossenen Gehäuse entfernt werden, ohne daß dabei die Wirksamkeit der angewandten Mikrowellen beeinträchtigt wird.

Aus der WO-A-8602842 ist schließlich eine Vorrichtung zum Sterilisieren von medizinischen Instrumenten oder Operationsinstrumenten bekannt, wobei auch andere Anwendungsfälle z.B. aus der Industrie angedeutet werden. In den Ausführungen zum Stand der Technik wird dann auch darauf hingewiesen, daß es bisher bekannt war, solche Instrumente in Sterilisationsflüssigkeiten oder durch Dampf zu sterilisieren. Um aus Kunststoff bestehende Instrumente nicht zu sehr zu beeinträchtigen, können keine zu hohen Temperaturen angewandt werden, und bei Anwendung von Sterilisationsflüssigkeiten können sich Luftblasen an den Instrumenten halten, in deren Bereich die Sterilisation möglicherweise unvollkommen ist.

Der vorliegenden Erfindung liegt die Aufgabe zugrunde, eine Vorrichtung vorzuschlagen, mit der eine besonders schonende, aber dennoch wirksame Behandlung von Stoffen in loser Form gewährleistet ist.

Gemäß der Erfindung ist die Vorrichtung der eingangs genannten Art dadurch gekennzeichnet, daß der Behälter als liegender, zylindrischer Behälter ausgebildet ist, an dessen beiden Stirnwänden ein Mischwerk zum Umwälzen der in dem Behälter lose liegenden Stoffe drehbar gelagert ist, und daß im mittleren Bereich der zylindrischen Wand des Behälters eine oder mehrere Mikrowellen-Einheiten außerhalb des Behälters angeordnet sind, deren Mikrowellenenergie über Einspeiseöffnungen in den Behälter von außen einspeisbar ist.

Als Mischwerk wird zweckmäßigerweise entweder ein Doppelschnecken-Gegenstrommischwerk verwendet, oder ein Schlagschaufel-Mischwerk.

Die Einspeisung der Mikrowellenenergie von außen hat den wesentlichen Vorteil, daß die Mikrowellen-Einheiten außerhalb des Behälters angeordnet sein können und somit nicht dem in dem Behälter herrschenden Druck oder Vakuum sowie dem eventuell nachteiligen Einfluß der zu behandelnden Stoffe ausgesetzt sind; darüber hinaus ist es möglich, den Innenraum des Behälters frei von Vorsprüngen und toten Ecken zu halten, in denen sich die zu behandelnden Stoffe sonst festsetzen könnten. Die Einspeiseöffnungen für die Mikrowellen-Einheiten können auf einfache Weise durch Verschlußstücke aus dielektrischem Material verschlossen sein, um den Behälter auch an dieser Stelle druck- und/oder vakuumdicht zu verschließen.

Das Mischwerk wird zweckmäßigerweise von außen mit wahlweise verschiedenen Geschwindigkeiten angetrieben, wobei die Lagerwelle des Mischwerks über druck- und/oder vakuumdichte Stopfbuchsen herausgeführt ist. Eine einfache Beschickung und Entleerung der zu behandelnden Stoffe in bzw. aus dem Behälter ist dann möglich, wenn der Behälter auf der Oberseite an einem Ende mit einer verschließbaren Einfüllöffnung und auf der Unterseite mit einem verschließbaren Entleerungsschacht versehen ist. Darüber hinaus ist es sinnvoll, seitliche, verschließbare Reinigungsöffnungen vorzusehen. Die Mikrowellen-Einheiten sind vorzugsweise auf der Oberseite im mittleren Bereich des Behälters angeordnet.

Die Bauweise mit Tragkonsolen zum Tragen der gesamten Vorrichtung hat den Vorteil, daß gemäß einer weiteren vorteilhaften Ausführungsform die Tragkonsolen auf vier Fußpunkten gelagert sein können, in denen Wägezellen untergebracht sind. Auf diese Weise ist es möglich, die zu behandelnden Stoffe in ihrer Menge zu bestimmen.

Für manche Handlungszwecke ist es gemäß einer weiteren Ausbildung der Erfindung zweckmäßig, bei der Anwendung von Druck zusätzlich Dampf in den geschlossenen Behälter einzuleiten. Zum Trocknen der Stoffe kann der Behälter vorzugsweise unter Vakuum setzbar sein, wodurch auf einfache Weise eine schonende Trocknung durchführbar ist.

Die Erfindung wird nachfolgend anhand eines Ausführungsbeispiels unter Bezug auf die beigefügten Zeichnungen näher erläutert.

Es zeigen:
- Fig. 1: eine Seitenansicht der erfindungsgemäßen Vorrichtung, und
- Fig. 2: einen Schnitt entlang der Linie II-II der Fig. 1.

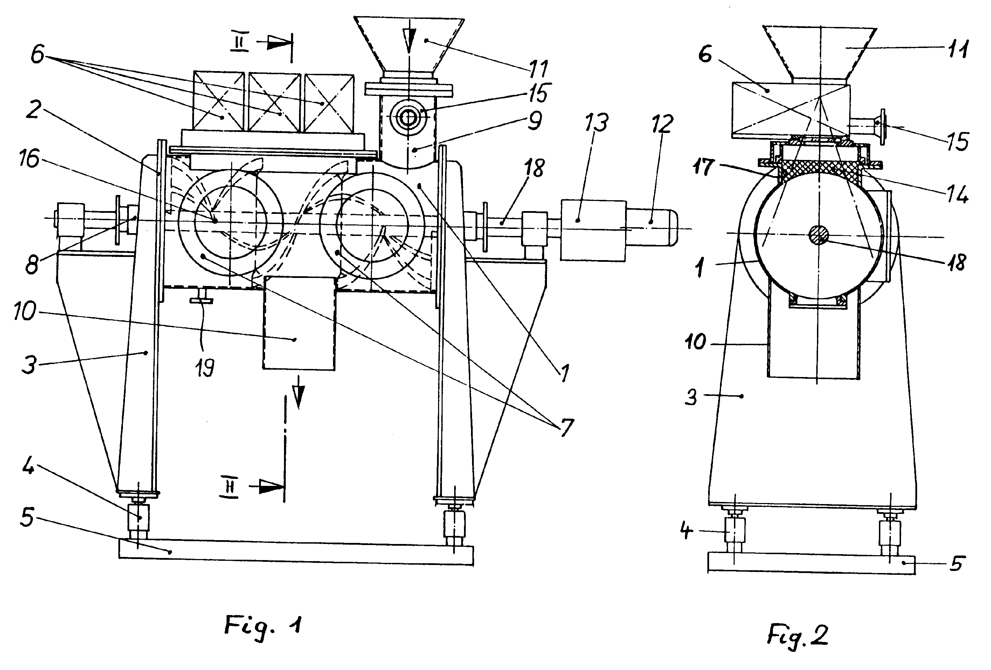

Die in den Fig. 1 und 2 gezeigte Vorrichtung enthält einen zylindrischen Behälter 1, der liegend angeordnet ist. An den Stirnwänden 2 sind Tragkonsolen 3 befestigt, die mittels vier Wägezellen 4 auf einem Grundrahmen 5 ruhen. Durch diese Wägezellen 4 ist es möglich, das Gewicht der gesamten Vorrichtung im leeren Zustand und im gefüllten Zustand zu wiegen und somit das Gewicht der eingefüllten Stoffe zu bestimmen. Auf der Oberseite des zylindrischen Behälters 1 sind mehrere Mikrowellen-Einheiten 6 vorgesehen, deren Mikrowellenenergie über Einspeiseöffnungen 17 in den zylindrischen Innenraum des Behälters 1 eingespeist werden können. Die Einspeiseöffnungen 17 sind durch Verschlußstücke 14 aus dielektrischem Material druck- und/oder vakuumdicht verschlossen.

An einem Endes des zylindrischen Behälters 1 befindet sich eine Einfüllöffnung 9 bzw. ein Einfüllstutzen, an den zum leichteren Einfüllen der zu behandelnden Stoffe ein Einfülltrichter 11 angeschlossen ist. An der Unterseite des Behälters 1 ist ein Entleerungsschacht 10 angeordnet, über den die behandelten Stoffe entleert werden können. Sowohl die Einfüllöffnung 9 als auch der Entleerungsschacht 10 sind im Betrieb verschließbar, im Falle des Entleerungsschachtes 10 durch eine entsprechende Verschlußklappe. Die zu behandelnden Stoffe liegen in loser Form vor, d.h. sie sind nicht in Behältern oder Gebinden enthalten.

Innerhalb des zylindrischen Behälters 1 ist ein Mischwerk 16 vorgesehen, das im vorliegenden Beispiel als Doppelschnecken-Gegenstrommischwerk ausgebildet ist, um eine wirksame Umwälzung der zu behandelnden Stoffe innerhalb des Behälters 1 zu ermöglichen. Dies führt zu einer besonders gleichmäßigen und schonenden Behandlung. Das Mischwerk 16 ist an den Stirnseiten des Behälters 1 gelagert, und die Lagerwelle 18 ist durch druck- und/oder vakuumdichte Stopfbuchsen herausgeführt. An das eine Ende der Lagerwelle 18 ist über ein Getriebe 13 ein Antriebsmotor 12 angeschlossen, mit dem das Mischwerk 16 mit geeigneter Geschwindigkeit angetrieben werden kann. Die Drehzahl des Mischwerkes 16 ist durch eine entsprechende Verstell- oder Regelvorrichtung variierbar.

Da der Behälter 1 allseits verschlossen ist, aber gelegentlich eine Reinigung oder Inspektion des Inneren, insbesondere des Mischwerkes 16, erforderlich werden kann, sind seitlich zwei Reinigungsöffnungen 7 vorgesehen, die über druck- und/oder vakuumdichte Verschlüsse verschlossen sind.

Am Einfüllstutzen der Einfüllöffnung 9 ist ein Vakuum/Druckanschluß 15 vorgesehen, über den das Innere des Behälters 1 unter Druck gesetzt oder evakuiert werden kann. Außerdem ist vorzugsweise im unteren Bereich ein Dampfanschluß 19 vorgesehen, über den beim Entkeimen oder Sterilisieren wahlweise auch Dampf eingeleitet werden kann, um das zu behandelnde Gut anzufeuchten und zusätzlich aufzuheizen.

Der Behälter 1 der erfindungsgemäßen Vorrichtung ist vorzugsweise sowohl druckdicht als auch vakuumdicht ausgebildet; dies gilt auch für die anderen Bauteile wie Stopfbuchsen, Verschlüsse usw. Mit einer solchen Vorrichtung können die zu behandelnden Stoffe dann wahlweise entweder unter Druck (Entkeimen bzw. Sterilisieren) oder Vakuum (sowohl Trocknen als auch Entkeimen oder Sterilisieren) behandelt werden, je nach Wirksamkeit und Verträglichkeit. Es ist jedoch auch möglich, den Behälter 1 nur druckdicht oder vakuumdicht auszuführen; in einem Fall ist nur die eine oder andere Behandlungsart möglich.

Es können statt des im Ausführungsbeispiel verwendeten Doppelschnecken-Gegenstrommischwerkes auch andere Mischwerksarten oder Umwälzvorrichtungen verwendet werden, z.B. ein Schlagschaufel-Mischwerk.

## Patentansprüche

1. Vorrichtung zum Keimfreimachen, Desinfizieren und/ oder Trocknen von schüttfähigen Stoffen in loser Form in einem geschlossenen, dichten Behälter unter Anwendung von Druck oder Vakuum zusammen mit Mikrowellenenergie und unter Umwälzung der Stoffe in dem Behälter,
dadurch gekennzeichnet, daß
der Behälter (1) als liegender, zylindrischer Behälter ausgebildet ist, an dessen beiden Stirnwänden (2) ein Mischwerk (16) zum Umwälzen der in dem Behälter lose liegenden Stoffe drehbar gelagert ist, und
daß im mittleren bereich der zylindrischen Wand des Behälters (1) eine oder mehrere Mikrowellen-Einheiten (6) außerhalb des Behälters angeordnet sind, deren Mikrowellenenergie über Einspeiseöffnungen (17) in den Behälter von außen einspeisbar ist.

2. Vorrichtung nach Anspruch 1,
dadurch gekennzeichnet, daß das Mischwerk (16) als Doppelschnecken-Gegenstrommischwerk ausgebildet ist.

3. Vorrichtung nach Anspruch 1,
dadurch gekennzeichnet, daß das Mischwerk (16) als Schlagschaufel-Mischwerk ausgebildet ist.

4. Vorrichtung nach einem der Ansprüche 1 - 3,
dadurch gekennzeichnet, daß die Einspeiseöffnungen (17) für die Mikrowellenenergie durch Verschlußstücke (14) aus dielektrischem Material verschlossen sind.

5. Vorrichtung nach einem der Ansprüche 1 - 4,
dadurch gekennzeichnet, daß das Mischwerk (16) von außen mit wahlweise verschiedenen Geschwindigkeiten antreibbar ist und die Lagerwelle (18) des Mischwerkes (16) über druck- und/oder vakuumdichte Stopfbuchsen (8) herausgeführt ist.

6. Vorrichtung nach einem der Ansprüche 1 - 5,
dadurch gekennzeichnet, daß der Behälter (1) in der zylindrischen Wand auf der Oberseite an einem Ende mit einer verschließbaren Einfüllöffnung (9) und auf der Unterseite mit einem verschließbaren Entleerungsschacht (10) versehen ist.

7. Vorrichtung nach einem der Ansprüche 1 - 6,
dadurch gekennzeichnet, daß in der zylindrischen Wand seitliche, verschließbare Reinigungsöffnungen (7) vorgesehen sind.

8. Vorrichtung nach einem der Ansprüche 1 - 7,
dadurch gekennzeichnet, daß im Bereich der Stirnwände (2) des Behälters (1) Tragkonsolen (3) zum Tragen der gesamten Vorrichtung befestigt sind.

9. Vorrichtung nach Anspruch 8,
dadurch gekennzeichnet, daß die Tragkonsolen (3) auf vier Fußpunkten gelagert sind, in denen Wägezellen (4) untergebracht sind.

10. Vorrichtung nach einem der vorstehenden Ansprüche unter Anwendung von Druck,
dadurch gekennzeichnet, daß zusätzlich Dampf in den geschlossenen Behälter einleitbar ist.

11. Vorrichtung nach einem der vorstehenden Ansprüche,
dadurch gekennzeichnet, daß zum Trocknen der Stoffe der Behälter unter Vakuum setzbar ist.

## Claims

1. Apparatus for sterilizing, disinfecting and/or drying of loose bulk material in a closed tight vessel using pressure or vacuum together with microwave energy whilst agitating the material in the vessel, characterized by the fact
that the vessel (1) is constructed as a horizontal, cylindrical container, where on its two front walls (2) a mixing mechanism (16) is located in rotary fit for agitating the material contained loosely in the vessel, and by the fact
that in the center part of the cylindrical wall of the vessel (1) one or more microwave units (6) are arranged outside the vessel and their microwave energy being fed into the vessel (1) from outside via injection openings (17).

2. Apparatus according to Claim 1,
characterized by the fact that the mixing mechanism (16) is constructed as a double-worm counterflow mixing mechanism.

3. Apparatus according to Claim 1,
characterized by the fact that the mixing mechanism (16) is constructed as an impeller mixing mechanism.

4. Apparatus according to one of the Claims 1 - 3,
characterized by the fact that the injection openings (17) for the microwave energy are closed off by closure pieces (14) made of dielectric material.

5. Apparatus according to one of the Claims 1 - 4,
characterized by the fact that the mixing mechanism (16) can be externally driven at selectably different speeds and that the bearing shaft (18) of the mixing mechanism (16) is brought out through pressure- and vacuum-tight stuffing boxes (8).

6. Apparatus according to one of the Claims 1 - 5,
characterized by the fact that the vessel (1) in its cylindrical wall is provided on the top side, at one end, with a closable filling opening (9) and on the underside with a closable emptying shaft (10).

7. Apparatus according to one of the Claims 1 - 6,
characterized by the fact that closable cleaning openings (7) are provided laterally in the cylindrical wall.

8. Apparatus according to one of the Claims 1 - 7,
characterized by the fact that brackets (3) are attached in way of the front walls (2) of the vessel (1) for supporting the overall apparatus.

9. Apparatus according to Claim 8,
characterized by the fact that the brackets (3) are kept on four feet in which weighing cells (4) are contained.

10. Apparatus according to one of the beforegoing Claims using pressure,
characterized by the fact that steam can additionally be fed into the closed vessel.

11. Apparatus according to one of the beforegoing Claims,
characterized by the fact that vacuum can be generated within the vessel for drying the bulk material.

## Revendications

1. Appareil pour la stérilisation, la désinfection et/ou le séchage des matières en vrac mobiles, qui se trouvent dans un récipient fermé, étanche, en employant de la pression ou du vide avec de l'énergie micro-onde, pendant que les matières dans le récipient sont agitées, caracterisé par le fait
que le récipient (1) est construit comme conteneur couché et cylindrique sur les deux parois frontales (2) duquel se trouve, en forme tournante, un dispositif de mélange (16) pour l'agitation des matières mobiles dans le récipient, et par le fait
qu'il y a une ou plusieurs unité micro-ondes (6) dans la partie centrale de la paroi cylindrique du récipient (1), disposées à l'extérieur du récipient et dont l'énergie micro-ondes peut être alimentée dans le récipient de l'extérieur via des ouvertures d'injection (17).

2. Appareil selon la revendication 1,
caracterisé par le fait que le dispositif de mélange (16) est construit comme dispositif de mélange à deux vis contre-courant.

3. Appareil selon la revendication 1,
caracterisé par le fait que le dispositif de mélange (16) est construit comme dispositif de mélange à pales.

4. Appareil selon l'une des revendications 1 à 3,
caracterisé par le fait que les ouvertures d'injection (17) pour l'énergie micro-ondes sont obturées par des pièces de fermeture (14) en matériel diélectrique.

5. Appareil selon l'une des revendications 1 à 4,
caracterisé par le fait que le dispositif de mélange (16) peut être actionné de l'extérieur, aux vitesses facultativement variées, et que l'arbre palier (18) du dispositif de mélange (16) est apporté en dehors par des presses-étoupes (8) étanches à la pression et au vide.

6. Appareil selon l'une des revendications 1 à 5,
caracterisé par le fait que le récipient (1) dans sa parois cylindrique est pourvu d'une ouverture de remplissement fermante (9) au côté supérieur, à une extrémité, et d'une cheminée de vidange (10) fermante au côté inférieur.

7. Appareil selon l'une des revendications 1 à 6,
caracterisé par le fait qu'il y a des ouvertures de nettoyage fermantes (7) disposées sur le côté dans la paroi cylindrique.

8. Appareil selon l'une des revendications 1 à 7,
caracterisé par le fait que des consoles portantes (3) sont attachées près des parois frontales (2) du récipient (1) pour porter l'appareil entier.

9. Appareil selon la revendication 8,
caracterisé par le fait que les consoles portantes (3) sont logées sur quatre pieds dans lesquels se trouvent des cellules de pesage (4).

10. Appareil selon l'une quelconque des revendications précédentes en employant de la pression,
caracterisé par le fait que, au surplus, de la vapeur peut être alimentée dans le récipient fermé.

11. Appareil selon l'une quelconque des revendications précédentes,
caracterisé par le fait que le récipient peut être mis sous vide pour le sèchage des matières.
